# EUROPEAN PATENT SPECIFICATION

(11) **EP 3 773 830 B1**
(45) Date of publication and mention of the grant of the patent: **13.03.2024**
(21) Application number: 19715349.7
(22) Date of filing: 20.03.2019
(51) Int. Cl.: A61M 15/00, A61M 11/00, B05B 17/06

(54) **SYSTEMS FOR PRODUCING AN AEROSOL**
SYSTEME ZUR HERSTELLUNG EINES AEROSOLS
SYSTÈMES DE PRODUCTION D'UN AÉROSOL

(30) Priority: 30.03.2018 US 201815942304
(43) Date of publication of application: 17.02.2021
(73) Proprietor: L'OREAL, 75008 Paris (FR); Miller, Zane Bowman Allen, Redmond WA 98052-4952 (US)
(72) Inventor: BESEN, Richard, Clark, NJ 07066 (US); MILLER, Zane Bowman Allen, Redmond, WA 98052 (US)
(74) Representative: Casalonga
(86) International application number: PCT/US2019/023231
(87) International publication number: WO 2019/190865

(56) References cited:
- EP-A1- 2 987 520
- EP-A1- 2 996 748
- US-A1- 2002 129 812
- US-B2- 6 851 626

## Description

### SUMMARY

In an aspect, the present disclosure provides a system comprising a nebulizing body including at least one electro-mechanical nebulizing element; and a replaceable cartridge including one or more fluid reservoirs, and a valve in fluid communication with the one or more fluid reservoirs. In some embodiments, the nebulizing body is configured to releaseably receive the replaceable cartridge.

This forgoing summary is provided to introduce a selection of concepts in a simplified form that are further described below in the Detailed Description. This summary is not intended to identify key features of the claimed subject matter, nor is it intended to be used as an aid in determining the scope of the claimed subject matter.

The present invention relates to a system according to the preamble of claim 1, such e.g. known from US 6851626B2 or EP 2987520A1.

### DESCRIPTION OF THE DRAWINGS

FIGURE 1A is a front view of a system in accordance with an embodiment of the disclosure;
FIGURE 1B is a top plan view of the system of FIGURE 1A;
FIGURE 1C is a partially-exploded perspective view of the system of FIGURE 1A;
FIGURE 1D is a cross-sectional view of the system of FIGURE 1A taken along lines 1D-1D in FIGURE 1A;
FIGURE 1E is an isometric cutaway view of the system of FIGURE 1A taken along lines 1E-1E in FIGURE 1B;
FIGURE 2A is a perspective view of the system of FIGURE 1C showing an aerosol discharged from the system;
FIGURE 2B is another cross-section view of the system of FIGURE 1A taken along the lines 1D-1D in FIGURE 1B showing an aerosol discharged from the system as a result of switch actuation;
FIGURE 2C is an isometric cutaway view of the system of FIGURE 2B taken along lines 2C-2C in FIGURE 2A;
FIGURE 3A is a perspective view of a replaceable cartridge in accordance with an embodiment of the disclosure;
FIGURE 3B is a cross-sectional view of the replaceable cartridge of FIGURE 3A taken along the lines 3B-3B in FIGURE 3A;
FIGURE 4 is an illustration of an electro-mechanical nebulizing assembly in accordance with an embodiment of the disclosure; and
FIGURE 5 is a block diagrammatic illustration of the internal operating structure of the system of FIGURE 1A and its associated internal assemblies in accordance with an embodiment of the disclosure.

Aspects and many of the attendant advantages of the claimed subject matter will become more readily appreciated as the same become better understood by reference to the following detailed description, when taken in conjunction with the accompanying drawings.

The detailed description set forth below in connection with the appended drawings, where like numerals reference like elements, is intended as a description of various embodiments of the disclosed subject matter and is not intended to represent the only embodiments. Each embodiment described in this disclosure is provided merely as an example or illustration and should not be construed as preferred or advantageous over other embodiments. The illustrative examples provided herein are not intended to be exhaustive or to limit the claimed subject matter to the precise forms disclosed. The invention is defined by the appended claims.

### DETAILED DESCRIPTION

Described herein are systems and methods for delivery of formulations in aerosol form onto skin. Certain conventional nebulizers couple with a cartridge containing a formulation for application onto skin. It would be advantageous to be able to use sequentially many cartridges containing various formulations with a single nebulizing body. However, many conventional nebulizers do not adjust the operation of nebulizing components according to specific characteristics of a formulation disposed in a cartridge coupled thereto, such as formulation viscosity. Accordingly, during operation such conventional nebulizers may fail to convert all or some of the formulation from a liquid to a mist of fine droplets, for example, because they provide insufficient power to the nebulizing components.

Further, many conventional nebulizers couple with cartridges containing formulations for application onto the skin in a way that permanently opens the cartridge. In this regard, formulation remaining in the cartridge may leak from one or more portions of the cartridge when the cartridge is uncoupled from the nebulizer. Accordingly, a user cannot replace a first cartridge that is not completely empty with another cartridge without remaining formulation leaking from the first cartridge. Additionally, any such remaining formulation maybe altered by, for example, exposure to air due to permanently opening the cartridge, thus rendering it unsuitable for future use.

To that end, the following discussion provides examples of systems including a nebulizing body including at least one electro-mechanical nebulizing element; and a replaceable cartridge including one or more fluid reservoirs; and a valve in fluid communication with the one or more fluid reservoirs, wherein the nebulizing body is configured to releaseably receive the replaceable cartridge. As will be described in more detail below, in an embodiment the valve is in a closed state when the replaceable cartridge is not received by and engaged with the nebulizing body. In that regard, a formulation disposed with the fluid reservoir will not leak or otherwise leave the fluid reservoir when the replaceable cartridge is not received by and engaged with the nebulizing body. Additionally, as discussed further herein, in an embodiment, the systems described herein include discharge aerosol circuitry operably coupled to the at least one electro-mechanical nebulizing element and configured to generate a discharge aerosol responsive to one or more inputs indicative of a replaceable cartridge identification. In this regard, in an embodiment the systems described herein are configured to operate the electro-mechanical nebulizing element according to a formulation disposed within the fluid reservoir to discharge an aerosol therefrom.

In the following description, numerous specific details are set forth in order to provide a thorough understanding of one or more embodiments of the present disclosure. It will be apparent to one skilled in the art, however, that many embodiments of the present disclosure may be practiced without some or all of the specific details. In some instances, well-known process steps have not been described in detail in order not to unnecessarily obscure various aspects of the present disclosure. Further, it will be appreciated that embodiments of the present disclosure may employ any combination of features described herein.

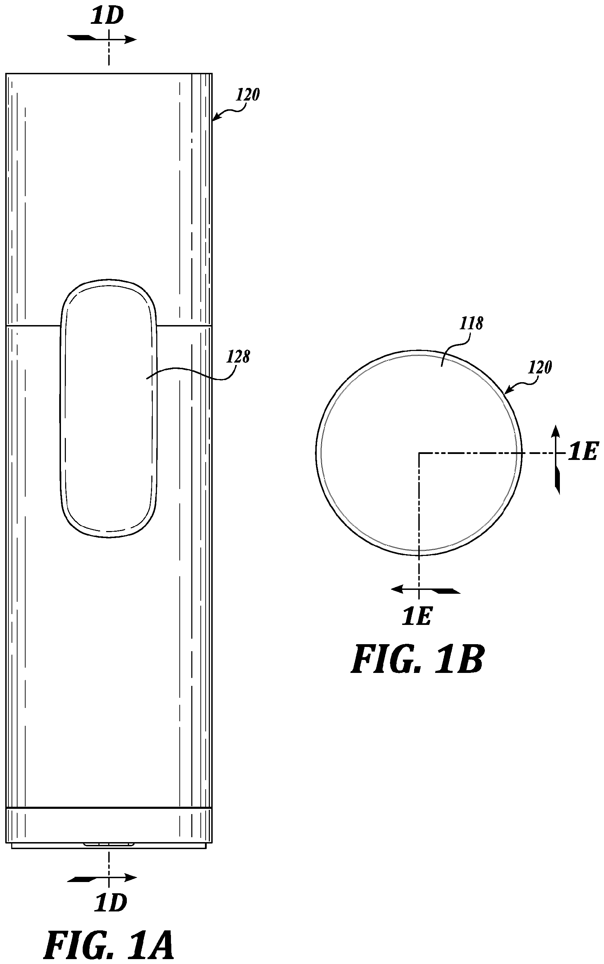

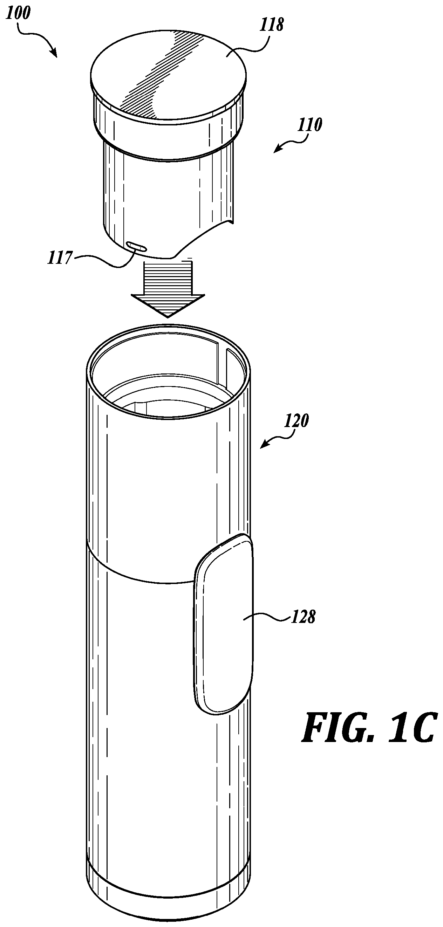

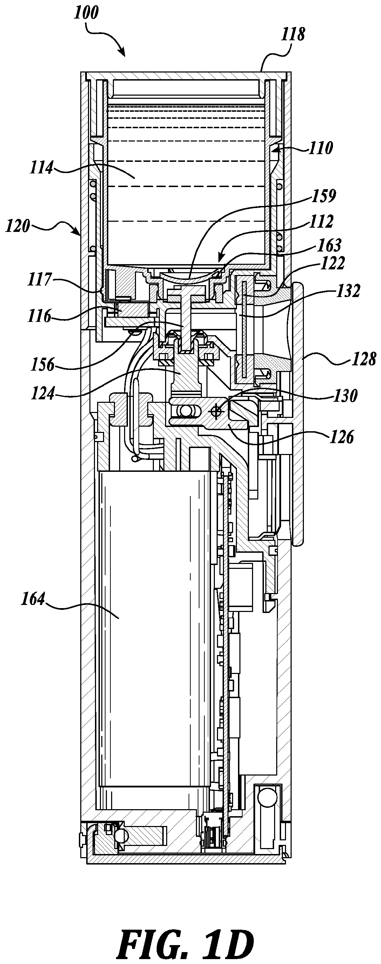

FIGURE 1A is a front view of a system 100 in accordance with an embodiment of the disclosure. FIGURE 1B is a top plan view of the system 100 of FIGURE 1A. FIGURE 1C is a partially-exploded perspective view of the system 100 of FIGURE 1A. FIGURE 1D is a cross-sectional view of the system 100 of FIGURE 1A taken along lines 1D-1D in FIGURE 1A. As shown in FIGURES 1A-1D, an embodiment of system 100 includes a nebulizing body 120 including a nebulizing assembly 122. The system 100 further includes a replaceable cartridge 110 including a fluid reservoir 114 and a valve 112 in fluid communication with the fluid reservoir 114. FIGURE 1C is a partially-exploded view of the system 100 illustrating one embodiment of the replaceable cartridge 110 uncoupled from nebulizing body 120.

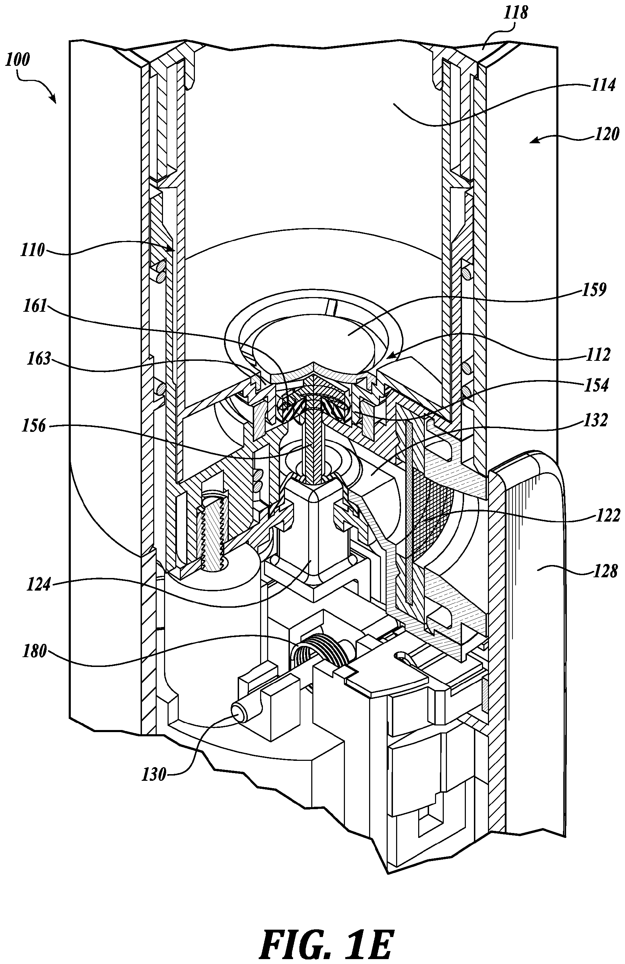

As illustrated in FIGURES 1A, 1B, 1D, and 1E, the replaceable cartridge 110 is releaseably received by the nebulizing body 120. As used herein, the replaceable cartridge 110 is releaseably received by the nebulizing body when the replaceable cartridge 110 is securely but removably coupled to the nebulizing body 120. In this regard and as discussed further herein, the replaceable cartridge 110 is releaseably received by the nebulizing body 120 such that the replaceable cartridge 110 can be securely coupled to and subsequently released by the nebulizing body 120 one or more times. In an embodiment, the replaceable cartridge 110 includes one or more detents 117 and the nebulizing body 120 includes detent-receiving structures, or vice versa, both of which are configured to cooperatively and removeably couple the replaceable cartridge 110 and the nebulizing body 120. Of course, the system 100 can include other cooperatively-coupling structures, such as threads configured to removeably couple the replaceable cartridge 110 and the nebulizing body 120.

In an embodiment, the nebulizing body 120 is configured to releaseably receive the replaceable cartridge 110 without engaging with the releasable cartridge 100, thus leaving the valve 112 in a closed state, as illustrated in FIGURE 1D. As used herein, the replaceable cartridge 110 is engaged with the nebulizing body 120 when the valve 112 is in an open state and the fluid reservoir 114 of the replaceable cartridge 110 is in fluid communication with the nebulizing assembly 122. Accordingly, the valve 112 is in a closed state when the replaceable cartridge 110 is not engaged with the nebulizing body 120. Thus, when the replaceable cartridge 110 is not engaged with the nebulizing body 120, the fluid reservoir 114 is not coupled in fluid communication with the nebulizing assembly 122. In this regard, the replaceable cartridge 110 in an unengaged configuration is received by the nebulizing body 120 and, accordingly, the valve 112 is in a closed state, as illustrated FIGURES 1D and 1E.

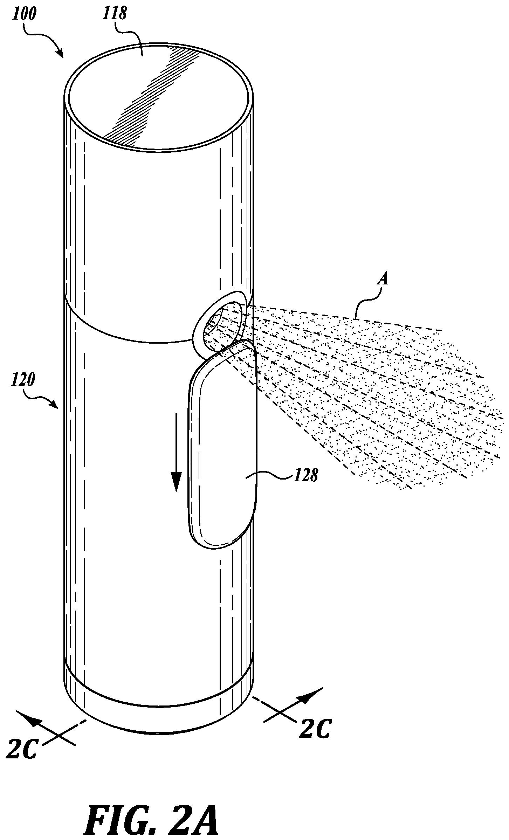

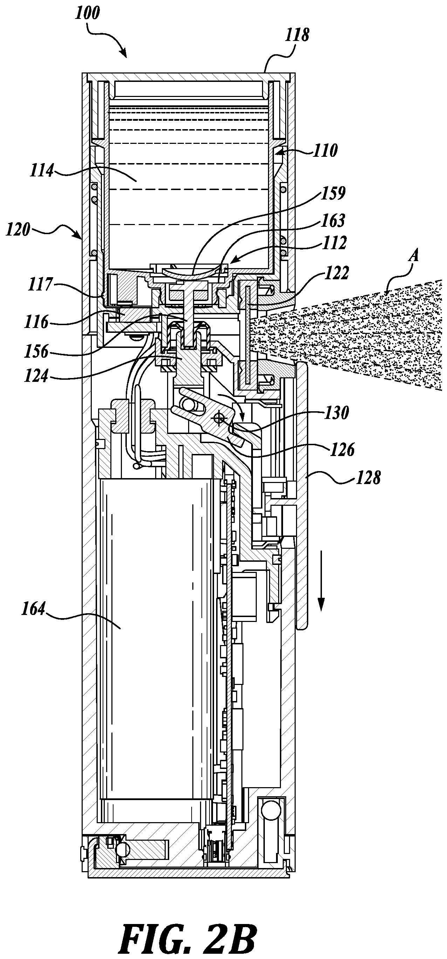

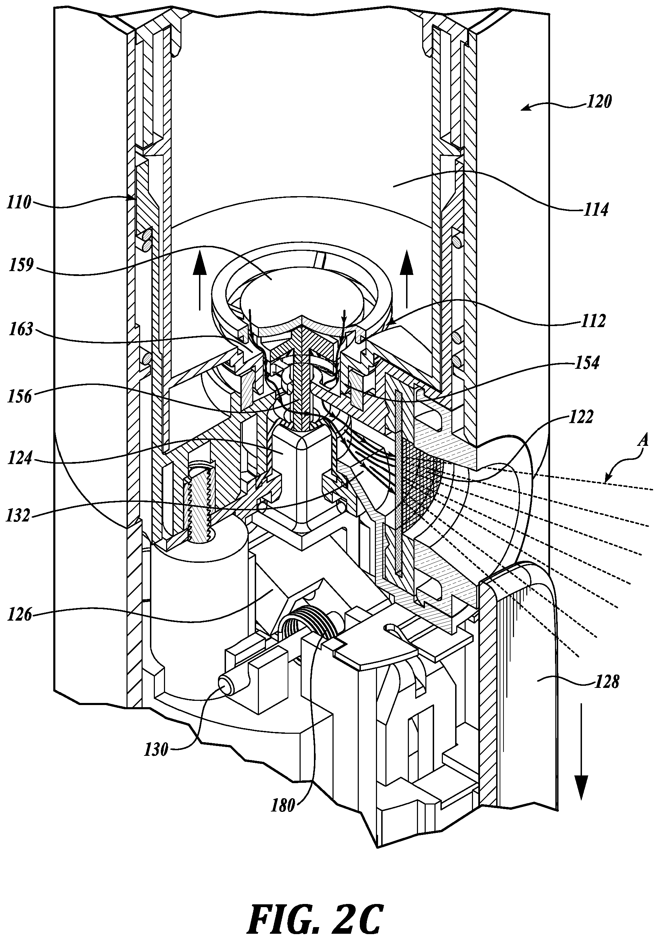

Alternatively, the valve 112 is in an open state and the fluid reservoir 114 is coupled in fluid communication with the at least one nebulizing assembly 122 when the replaceable cartridge 110 is received by and engaged with the nebulizing body 120. In this regard, attention is directed to FIGURES 2A, 2B, and 2C, in which a configuration of the system 100 according to embodiments of the disclosure is illustrated. Referring to FIGURE 2B and 2C, the replaceable cartridge 110 is shown releaseably received by the nebulizing body 120 by detents 117. In order to be engaged, the nebulizing body 120 in some embodiments includes an actuatable switch 128 configured to selectively place the valve 112 in an open state.

For example, as illustrated in FIGURES 1D, 1E, 2B, and 2C, the nebulizing body 120 includes a switch 128 operatively coupled to a cam 126, which is, in turn, operatively coupled to a push rod 124. In this embodiment, the cam 126 pivots about pivot 130 against a biasing force of a spring 180. In an embodiment, the spring 180 biases the cam 126 into a first position in which the valve 112 in a closed position, as illustrated in FIGURES 1D and 1E. In use, as the switch 128 is selectively actuated via movement (e.g., linear movement, etc.), such as by the hand of a user, from the first position, as illustrated in FIGURE 1D and 1E, to a second position, as illustrated in FIGURES 2B and 2C, the cam 126 is rotated (in the direction of the arrow) against the biasing force of the spring 180. Rotation of the cam 126 causes the push rod 124 to be moved (e.g., linearly, etc.) to place the valve 112 in an open state. In this regard, the valve 112 is placed in an open state and the fluid reservoir 114 is coupled in fluid communication with the nebulizing assembly 122 when the switch 128 is activated, such as by the hand of a user. Removal of the hand of the user from the switch 128 allows the biasing force of the spring 180 to return the cam 126 and the switch 128 the non-engaged position (e.g., unactuated position, etc.) as shown in FIGURES 1D and 1E

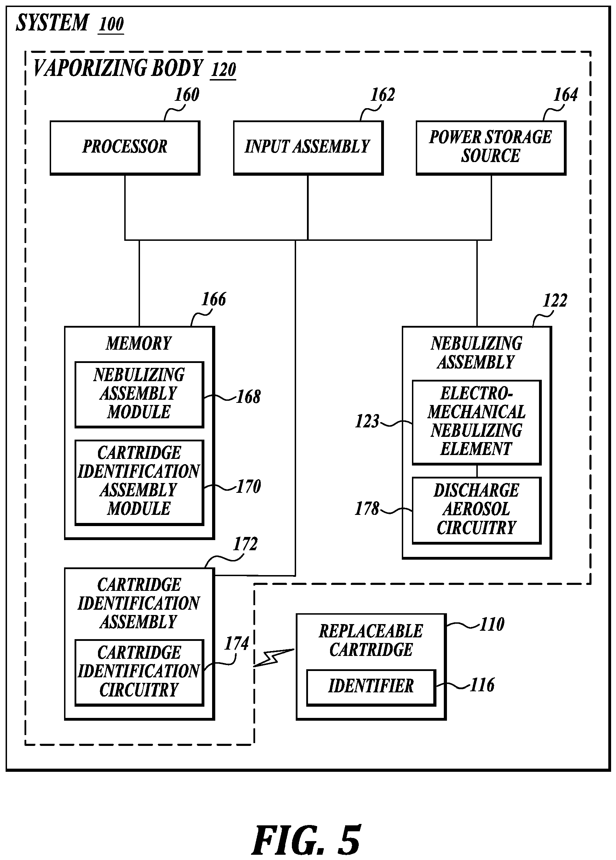

In an embodiment, when the switch 128 is activated the valve 112 is placed in an open state and the discharge aerosol circuitry (discussed further herein with respect to FIGURE 5), which is operably coupled to the nebulizing assembly 122, is activated, thereby causing an aerosol A to be discharged from the system 100, as illustrated in FIGURES 2A, 2B, and 2C. As illustrated in FIGURE 2C, in an embodiment, when the valve 112 in an open state, fluid in the fluid reservoir 114 is allowed to flow past valve seal 159 and into a space 132 that is disposed in fluid contact with the nebulizing assembly 122. In this regard, an aerosol A is discharged by the system 100 when the valve 112 is in an open state. This is in contrast to the embodiment illustrated in FIGURE 1D, in which the valve seal 159 is shown disposed against the valve seat 163, thereby placing the valve 112 in a closed state. In an embodiment, element 161 forms part of a valve seal. In an embodiment, element 161 forms part of a valve stop.

In an embodiment, the system 100 includes an input assembly, such as input assembly 162 (see FIGURE 5) in addition to switch 128. As discussed further herein with respect to FIGURE 5, in an embodiment, the discharge aerosol circuitry is triggered (e.g., receive an input, electronically actuated, energized, etc.) to activate the nebulizing assembly 122 from the input assembly, thereby discharging aerosol A from system 100. In this regard, the fluid reservoir 114 is placed in fluid communication with the nebulizing assembly 122 through activation of the switch 128 and the nebulizing assembly 122 is activated in response to activation of the input assembly.

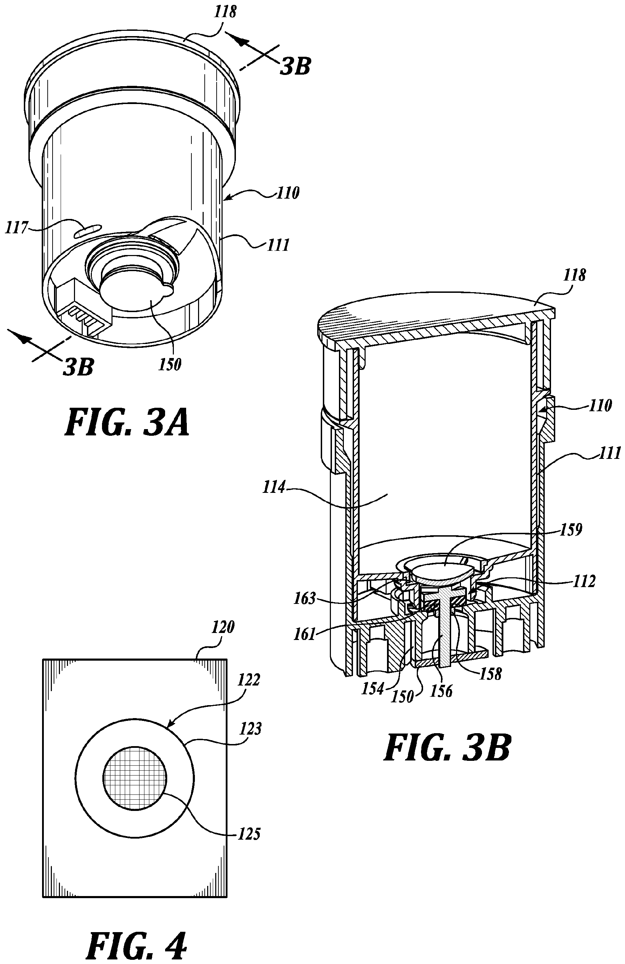

Referring to FIGURES 3A and 3B, the replaceable cartridge 110 including valve 112 is illustrated separately from the nebulizing body 120. The replaceable cartridge 110 is illustrated to include cartridge body 111 and a cartridge top 118, which, when coupled, define in part the fluid reservoir 114. In the illustrated embodiment, the valve 112 includes stop 161 coupled to shaft 156 and configured to limit the movement of shaft 156. In the illustrated embodiment, valve 112 also includes guide 158 slideably disposed around a portion of shaft 156 and configured to guide the movement of shaft 156 as it travels in opening and closing the valve 112. In FIGURES 3A and 3B, the replaceable cartridge 110 is shown to include protective seal 150, such a paper or plastic protective seal, configured to be removed by a user prior to use or punctured by coupling the removable cartridge 110 to the nebulizing body 120.

In operation, when the replaceable cartridge 110 is received by the nebulizing body 120 and, for example, the switch 128 has been activated (e.g., moved linearly, displaced, engaged, etc.), the push rod 124 displaces a valve seal 159 from the valve seat 163, thereby placing the valve 112 in an open state, as illustrated in FIGURE 2C. Referring to FIGURE 3A, the valve 112 is shown to include an annular ring 154 configured to cooperatively engage with nebulizing body 120 to provide a fluid-tight seal between the replaceable cartridge 110 and space 132 disposed within nebulizing body 120 (See FIGURE 2C). In this regard, the valve 112 is configured to direct any fluid in the fluid reservoir 114 to the nebulizing assembly 122. Further, when the replaceable cartridge 110 is engaged with and received by the nebulizing body 120 and, accordingly, the valve 112 is an open state, fluid from the replaceable cartridge 110 is prevented from entering other portions of the nebulizing body 120.

It will be appreciated that valve 112 can be any valve configured to selectively place fluid reservoir 114 in fluid communication with nebulizing assembly 122 when the replaceable cartridge 110 is received by and engaged with the nebulizing body 120. In an embodiment, the valve 112 operates as a one-way valve, such as a check valve. In an embodiment, the check valve can be of the ball type, the diaphragm type, or of the swing type. In an embodiment, valve 112 is a two-way valve.

In an embodiment, the replaceable cartridge 110 includes a fluid disposed in the fluid reservoir 114. In an embodiment, the replaceable cartridge 110 includes a cosmetic or dermatological formulation disposed in the fluid reservoir 114. In an embodiment, the cosmetic or dermatological formulation includes a composition chosen from a foundation, a perfume, a moisturizer, a self-tanning agent, a lotion for the body or the face, a composition containing a hair agent, a sunscreen composition, and combinations thereof, among others.

In an embodiment, the system 100 includes two or more replaceable cartridges 110 each configured to be received by and engage with the nebulizing body 120. In an embodiment, each of the two or more replaceable cartridges 110 includes an identifier 116 configured to emit an input for receipt by cartridge identification circuitry (See FIGURE 5). The input is indicative of the formulation disposed in the respective fluid reservoirs 114.

Referring to FIGURE 4, a nebulizing assembly 122 formed in accordance with an exemplary embodiment of the present disclosure and configured for use with system 100 will now be described in detail. As noted above, the nebulizing assembly 122 is configured to produce on-demand aerosol. The terms "nebulize," "nebulizing," etc., should be construed to include atomizing, misting, generating an aerosol, reducing to fine particles or spray, etc.

As shown in FIGURE 4, the nebulizing assembly 122 generally includes at least one electro-mechanical nebulizing element 123, such as a piezoelectric element, fluidically coupled to the fluid reservoir 114 (FIGURE 1D) when the valve 112 is in an open state and configured to discharge an aerosol (See FIGURES 2A-2C). In an embodiment, the nebulizing assembly 122 includes a mesh 125, such as a metallic mesh, disposed adjacent to the electro-mechanical nebulizing element 123, which receives a fluid from the fluid reservoir 114 of the replaceable cartridge 112. In the illustrated embodiment, the electro-mechanical nebulizing element 123 concentrically surrounds the mesh 125. In an embodiment, the nebulizing assembly 122 is a vibrating mesh dispenser assembly including a mesh 125 including apertures that is concentrically surrounded by the electro-mechanical nebulizing element 123. In an embodiment, the nebulizing assembly 122 is configured to discharge an aerosol including particles having an average diameter of less than 100 nm.

In an embodiment, the nebulizing assembly 122 is received within the replaceable cartridge 110. In an embodiment, the nebulizing assembly 122 is received within the nebulizing body 120. In an embodiment, the nebulizing assembly 122 includes replaceable nebulizing elements 123.

In an embodiment, during use, the electro-mechanical nebulizing element 123 contracts and expands upon the application of an alternating electric current, and consequently, the electro-mechanical nebulizing element 123 vibrates. When a liquid, such as a formulation from the fluid reservoir 114 of the replaceable cartridge 110, is in contact with the vibrating mesh 125, pressure builds in the vicinity of the mesh 125, creating a pumping action that extrudes the formulation through the apertures. When the mesh 125 vibrates at a sufficient frequency and with sufficient power, an aerosol A is formed from the formulation, which is emitted from the system 100 through mesh 125.

In an embodiment, the system 100 includes suitable circuitry for identifying one or more replaceable cartridges 110 received by the nebulizing body 120 and suitable circuitry for sending at least one output signal indicative of the identified replaceable cartridge(s) 110 for controlling and/or activating the nebulizing assembly 122. In that regard, attention is directed back to FIGURE 2B, where a system 100 including circuitry for identifying one or more replaceable cartridges in accordance with embodiments of the present disclosure is illustrated. In the illustrated embodiment, replaceable cartridge 110 includes an identifier 116. The nebulizing body 120 includes cartridge identification assembly 172 (not shown in FIGURE 2B). As discussed further herein with respect to FIGURE 5, in certain embodiments the identifier 116 includes circuitry configured to generate one or more inputs for receipt by the cartridge identification circuitry disposed within the cartridge identification assembly 172.

An embodiment of the internal operating structure of the system 100 and its associated internal assemblies is shown in block diagrammatic form in FIGURE 5. An exemplary operating structure of the system 100 includes a programmed microcontroller or processor 160 configured to control the delivery of power to the nebulizing assembly 122 from the power storage source 164. Further, the processor 160 is configured to operate in accordance with program instructions stored in memory 166 or otherwise stored in hardware format for controlling aspects of the nebulizing assembly 122.

In the embodiment shown, the nebulizing assembly 122 includes discharge aerosol circuitry 178 operably coupled to the electro-mechanical nebulizing element 123 and configured to activate the electro-mechanical nebulizing element 123 to generate a discharge aerosol A (See FIGURES 2A-2C). In one embodiment, the electro-mechanical nebulizing element 123 includes a piezoelectric element that defines part of the nebulizing assembly 122. The discharge aerosol circuitry 178 is configured to activate the nebulizing assembly 122 in response to inputs received from the processor 160, inputs received from one or more modules stored in the memory 166, such as the nebulizing assembly module 168 and the cartridge identification assembly module 170, and/or inputs received from other assemblies (such as the input assembly 162 and the identifier 116). In an embodiment the nebulizing assembly module 168 and the cartridge identification assembly module 170 includes any suitable programs, files, or instructions for activating and controlling the nebulizing assembly122.

The discharge aerosol circuitry 178 can be configured to receive input from an input assembly 162 (which may include an on/off button, a power adjust button, a mode control button, a nebulizing button, etc.). In an embodiment, the input assembly 162 is configured and arranged to selectively deliver power from the power storage source 164 to the nebulizing assembly 122, thereby generating aerosol A.

In an embodiment, the system 100 includes discharge aerosol circuitry 178 operably coupled to the nebulizing assembly 122 and configured to generate a discharge aerosol A responsive to one or more inputs indicative of a replaceable cartridge 110 identification. In this regard, in an embodiment, the system 100 includes a cartridge identification assembly 172 including cartridge identification circuitry 174, operably coupled to the discharge aerosol circuitry 178. In this regard, the cartridge identification assembly 172 is configured to receive one or more inputs from an identifier 116 carried by the replaceable cartridge 110 and to generate the one or more inputs indicative of a replaceable cartridge 110 identification for receipt by the cartridge identification circuitry 174.

In an embodiment, the one or more inputs indicative of a replaceable cartridge 110 identification correspond to a fluid disposed in the fluid reservoir 114, and wherein the discharge aerosol circuitry 178 is configured to operate the nebulizing assembly 122 based on the fluid disposed in the fluid reservoir 114. In this regard, in an embodiment, the system 100 is configured to operate the nebulizing assembly 122 based on, for example, a viscosity of a formulation disposed in the fluid reservoir 114. For example, an embodiment, the nebulizing assembly module 168 includes instructions configured to control the delivery of power to the nebulizing assembly 122 from the power storage source 164 controlling an oscillation frequency and an oscillation amplitude of the electro-mechanical nebulizing element 123 tailored to the viscosity of the particular fluid in the fluid reservoir 114. In this regard, sufficient power is provided to the nebulizing assembly 122 to nebulize the formulation and emit an aerosol A from the system 100.

In an embodiment, the cartridge identification assembly 172 is operatively coupled to the replaceable cartridge 110 by one or more of a wired connection or a wireless connection. In an embodiment, the wireless connection is a direct wireless connection, such as a Bluetooth connection, a near field communication (NFC) connection, a direct WiFi connection, or any other direct wireless connection. In an embodiment, the cartridge identification circuitry 174 includes one of a reader of a radiofrequency identification device and a near-field communications initiator and the replaceable cartridge 110 includes corresponding circuitry configured to communicate with the cartridge identification circuitry 174.

In an embodiment, the identifier 116 includes a portion configured to couple with the cartridge identification circuitry 174 having an electrical resistance indicative of the replaceable cartridge 110. In an embodiment, the identifier 116 includes one or more mechanical features indicative of the replaceable cartridge 110 and configured to engage with one or more switches included in the cartridge identification circuitry 174.

Certain embodiments disclosed herein utilize circuitry in order to implement treatment protocols, operably couple two or more components, generate information, determine operation conditions, control an appliance or method, process signals, and/or the like. Circuitry of any type can be used. In an embodiment, circuitry includes, among other things, one or more computing devices such as a processor (e.g., a microprocessor), a central processing unit (CPU), a digital signal processor (DSP), an application-specific integrated circuit (ASIC), a field-programmable gate array (FPGA), or the like, or any combinations thereof, and can include discrete digital or analog circuit elements or electronics, or combinations thereof. In an embodiment, circuitry includes one or more ASICs having a plurality of predefined logic components. In an embodiment, circuitry includes one or more FPGA having a plurality of programmable logic components.

In an embodiment, circuitry includes hardware circuit implementations (e.g., implementations in analog circuitry, implementations in digital circuitry, and the like, and combinations thereof). In an embodiment, circuitry includes combinations of circuits and computer program products having software or firmware instructions stored on one or more computer readable memories that work together to cause a device to perform one or more methodologies or technologies described herein. In an embodiment, circuitry includes circuits, such as, for example, microprocessors or portions of microprocessor, that require software, firmware, and the like for operation. In an embodiment, circuitry includes an implementation comprising one or more processors or portions thereof and accompanying software, firmware, hardware, and the like. In an embodiment, circuitry includes a baseband integrated circuit or applications processor integrated circuit or a similar integrated circuit in a server, a cellular network device, other network device, or other computing device. In an embodiment, circuitry includes one or more remotely located components. In an embodiment, remotely located components are operably coupled via wireless communication. In an embodiment, remotely located components are operably coupled via one or more receivers, transmitters, transceivers, or the like.

In an embodiment, circuitry includes one or more memory devices that, for example, store instructions or data. Non-limiting examples of one or more memory devices include volatile memory (e.g., Random Access Memory (RAM), Dynamic Random Access Memory (DRAM), or the like), non-volatile memory (e.g., Read-Only Memory (ROM), Electrically Erasable Programmable Read-Only Memory (EEPROM), Compact Disc Read-Only Memory (CD-ROM), or the like), persistent memory, or the like. Further non-limiting examples of one or more memory devices include Erasable Programmable Read-Only Memory (EPROM), flash memory, or the like. The one or more memory devices can be coupled to, for example, one or more computing devices by one or more instructions, data, or power buses.

In an embodiment, circuitry of the system 100 includes a computer-readable media drive or memory slot configured to accept signal-bearing medium (e.g., computer-readable memory media, computer-readable recording media, or the like). In an embodiment, a program for causing a system to execute any of the disclosed methods can be stored on, for example, a computer-readable recording medium (CRMM), a signal-bearing medium, or the like. Non-limiting examples of signal-bearing media include a recordable type medium such as any form of flash memory, magnetic tape, floppy disk, a hard disk drive, a Compact Disc (CD), a Digital Video Disk (DVD), Blu-Ray Disc, a digital tape, a computer memory, or the like, as well as transmission type medium such as a digital and/or an analog communication medium (e.g., a fiber optic cable, a waveguide, a wired communications link, a wireless communication link (e.g., transmitter, receiver, transceiver, transmission logic, reception logic, etc.). Further non-limiting examples of signal-bearing media include, but are not limited to, DVD-ROM, DVD-RAM, DVD+RW, DVD-RW, DVD-R, DVD+R, CD-ROM, Super Audio CD, CD-R, CD+R, CD+RW, CD-RW, Video Compact Discs, Super Video Discs, flash memory, magnetic tape, magneto-optic disk, MINIDISC, non-volatile memory card, EEPROM, optical disk, optical storage, RAM, ROM, system memory, web server, or the like.

It should be noted that for purposes of this disclosure, terminology such as "upper," "lower," "vertical," "horizontal," "inwardly," "outwardly," "inner," "outer," "front," "rear," etc., should be construed as descriptive and not limiting the scope of the claimed subject matter. Further, the use of "including," "comprising," or "having" and variations thereof herein is meant to encompass the items listed thereafter and equivalents thereof as well as additional items. Unless limited otherwise, the terms "connected," "coupled," and "mounted" and variations thereof herein are used broadly and encompass direct and indirect connections, couplings, and mountings. The term "about" means plus or minus 5% of the stated value.

The principles, representative embodiments, and modes of operation of the present disclosure have been described in the foregoing description. However, aspects of the present disclosure which are intended to be protected are not to be construed as limited to the particular embodiments disclosed. Further, the embodiments described herein are to be regarded as illustrative rather than restrictive. It will be appreciated that variations and changes may be made by others, and equivalents employed, without departing from the present disclosure. Accordingly, it is expressly intended that all such variations, changes, and equivalents fall within the scope of the present disclosure, as claimed.

## Claims

1. A system (100) comprising:
a nebulizing body (120) including at least one electro-mechanical nebulizing element (123); and
a replaceable cartridge (110) including one or more fluid reservoirs (114) and a valve in fluid communication with the one or more fluid reservoirs (114),
wherein the nebulizing body (120) is configured to releasably receive the replaceable cartridge, **characterized in that**
the nebulizing body (120) includes a switch configured to selectively engage the replaceable cartridge (110) when the replaceable cartridge is releasably received by the nebulizing body (120), thereby placing the valve in an open state.

2. The system (100) of Claim 1, wherein the valve is in an open state and the one or more fluid reservoirs (114) are coupled in fluid communication with the at least one electro-mechanical nebulizing element (123) when the replaceable cartridge (110) is received by and engaged with the nebulizing body (120).

3. The system (100) of Claim 1, wherein the valve is in a closed state and the one or more fluid reservoirs (114) are not coupled in fluid communication with the at least one electro-mechanical nebulizing element (123) in an unengaged configuration.

4. The system (100) of Claim 1, wherein nebulizing body (120) is configured to releasably receive the replaceable cartridge (110) without engaging with the replaceable cartridge.

5. The system (100) of Claim 1, wherein the nebulizing body (120) and the replaceable cartridge (110) are cooperatively configured such that the replaceable cartridge can be releasably received by and engaged with the nebulizing body (120) after having been previously releasably received by and engaged with the nebulizing body (120).

6. The system (100) of Claim 1, further comprising discharge aerosol circuitry (178) operably coupled to the at least one electro-mechanical nebulizing element (123) and configured to generate a discharge aerosol responsive to one or more inputs indicative of a replaceable cartridge identification (172), wherein the one or more inputs indicative of a replaceable cartridge identification (172) corresponds to one or more fluids disposed in the one or more fluid reservoirs (114), and wherein the discharge aerosol circuitry (178) is configured to operate the at least one electro-mechanical nebulizing element (123) based on the one or more fluids disposed in the one or more fluid reservoirs (114).

7. The system (100) of Claim 6, further comprising cartridge identification circuitry (178) operably coupled to the discharge aerosol circuitry (178), the cartridge identification circuitry (174) configured to receive one or more inputs from an identifier (116) carried by the replaceable cartridge and to generate the one or more inputs indicative of a replaceable cartridge identification (174) for receipt by the aerosol discharge circuitry (178),
wherein the cartridge identification circuitry (174) includes one of a reader of a radiofrequency identification device and a near-field communications initiator,
wherein the replaceable cartridge includes an identifier (116) configured to receive one or more inputs from the cartridge identification circuitry (174) and generate the one or more inputs indicative of a replaceable cartridge identification (172) for receipt by the cartridge identification circuitry (174).

8. The system (100) of Claim 7, wherein the identifier (116) includes a portion configured to couple with the cartridge identification circuitry (174) having an electrical resistance indicative of the replaceable cartridge.

9. The system (100) of Claim 7, wherein the identifier (116) includes one or more mechanical features indicative of the replaceable cartridge configured to engage with one or more switches included in the cartridge identification circuitry (174).

10. The system (100) of Claim 6, wherein the replaceable cartridge (110) is a first replaceable cartridge, the system further comprising a second replaceable cartridge (110) including one or more fluid reservoirs (114) and configured to be releasably received by the nebulizing body (120); a valve in fluid communication with the one or more fluid reservoirs (114); and a second identifier (116) configured to generate one or more inputs indicative of a second replaceable cartridge identification (172),
wherein the one or more inputs indicative of a second replaceable cartridge identification (172) corresponds to one or more fluids disposed in the one or more fluid reservoirs (114) of the second replaceable cartridge (110), and wherein the discharge aerosol circuitry (178) is configured to operate the at least one electro-mechanical nebulizing element (123) based on the one or more fluids disposed in the one or more fluid reservoirs (114) of the second replaceable cartridge (110).

11. The system (100) of Claim 1, wherein the replaceable cartridge (110) includes a cosmetic or dermatological composition disposed in the one or more fluid reservoirs (114).

12. The system (100) of Claim 1, wherein the at least one electro-mechanical nebulizing element (123) includes a piezoelectric element configured to be in fluid communication with the one or more fluid reservoirs (114) of the replaceable cartridge (110) when the valve is in an open state.

13. The system (100) of Claim 12, wherein the at least one electro-mechanical nebulizing element (123) further includes a mesh (125) disposed adjacent to the piezoelectric element and separated from the piezoelectric element by a space configured to receive a fluid from the one or more fluid reservoirs (114) of the replaceable cartridge (110).

14. The system (100) of Claim 1, wherein the at least one electro-mechanical nebulizing element (123) is configured to discharge an aerosol including particles having an average diameter of less than 100 µm.

15. The system (100) of Claim 12, further comprising discharge aerosol circuitry (178) operably coupled to the at least one electro-mechanical nebulizing element (123) including the piezoelectric element and configured to apply an electrical field to the piezoelectric element, the electrical field having a frequency and an amplitude responsive to one or more inputs indicative of a replaceable cartridge identification (172).

## Patentansprüche

1. System (100), Folgendes umfassend:
einen Vernebelungskörper (120), der wenigstens ein elektromechanisches Vernebelungselement (123) umfasst; und
eine auswechselbare Kartusche (110), die ein oder mehrere Fluidreservoire (114) und ein Ventil, das in Fluidverbindung mit dem einen oder den mehreren Fluidreservoiren (114) steht, umfasst,
wobei der Vernebelungskörper (120) dazu ausgestaltet ist, die auswechselbare Kartusche lösbar aufzunehmen, **dadurch gekennzeichnet, dass**
der Vernebelungskörper (120) einen Schalter umfasst, der dazu ausgestaltet ist, selektiv mit der auswechselbaren Kartusche (110) in Eingriff zu gelangen, wenn die auswechselbare Kartusche durch den Vernebelungskörper (120) lösbar aufgenommen ist, und dadurch das Ventil in einen offenen Zustand zu versetzen.

2. System (100) nach Anspruch 1, wobei das Ventil in einem offenen Zustand ist und das eine oder die mehreren Fluidreservoire (114) in Fluidverbindung mit dem wenigstens einen elektromechanischen Vernebelungselement (123) gekoppelt sind, wenn die auswechselbare Kartusche (110) durch den Vernebelungskörper (120) aufgenommen ist und mit diesem in Eingriff steht.

3. System (100) nach Anspruch 1, wobei in einer Nichteingriffskonfiguration das Ventil in einem geschlossenen Zustand ist und das eine oder die mehreren Fluidreservoire (114) nicht in Fluidverbindung mit dem wenigstens einen elektromechanischen Vernebelungselement (123) gekoppelt sind.

4. System (100) nach Anspruch 1, wobei der Vernebelungskörper (120) dazu ausgestaltet ist, die auswechselbare Kartusche (110) lösbar aufzunehmen, ohne mit der auswechselbaren Kartusche in Eingriff zu gelangen.

5. System (100) nach Anspruch 1, wobei der Vernebelungskörper (120) und die auswechselbare Kartusche (110) zusammenwirkend derart ausgestaltet sind, dass die auswechselbare Kartusche durch den Vernebelungskörper (120) lösbar aufgenommen werden und mit diesem in Eingriff gelangen kann, nachdem sie bereits zuvor durch den Vernebelungskörper (120) lösbar aufgenommen wurde und mit diesem in Eingriff stand.

6. System (100) nach Anspruch 1, das ferner eine Abgabeaerosolschaltungsanordnung (178) umfasst, die mit dem wenigstens einen elektromechanischen Vernebelungselement (123) wirkgekoppelt und dazu ausgestaltet ist, als Reaktion auf eine oder mehrere Eingaben, die eine der auswechselbaren Kartusche zugehörige Identifikation (172) angeben, ein Abgabeaerosol zu erzeugen, wobei die eine oder mehreren Eingaben, die eine der auswechselbaren Kartusche zugehörige Identifikation (172) angeben, einem oder mehreren Fluiden entsprechen, die in dem einen oder den mehreren Fluidreservoiren (114) angeordnet sind, und wobei die Abgabeaerosolschaltungsanordnung (178) dazu ausgestaltet ist, das wenigstens eine elektromechanische Vernebelungselement (123) basierend auf dem einen oder den mehreren Fluiden, die in dem einen oder den mehreren Fluidreservoiren (114) angeordnet sind, zu betreiben.

7. System (100) nach Anspruch 6, das ferner eine Kartuschenidentifikationsschaltungsanordnung (178) umfasst, die mit der Abgabeaerosolschaltungsanordnung (178) wirkgekoppelt ist, wobei die Kartuschenidentifikationsschaltungsanordnung (174) dazu ausgestaltet ist, eine oder mehrere Eingaben von einer Identifizierungseinrichtung (116), die von der auswechselbaren Kartusche getragen wird, zu empfangen und die eine oder mehreren Eingaben, die eine der auswechselbaren Kartusche zugehörige Identifikation (174) angeben, zum Empfang durch die Aerosolabgabeschaltungsanordnung (178) zu erzeugen,
wobei die Kartuschenidentifikationsschaltungsanordnung (174) eines aus einem Leser einer Funkfrequenzidentifikationsvorrichtung und einer Nahbereichskommunikationsinitiierungseinrichtung umfasst,
wobei die auswechselbare Kartusche eine Identifizierungseinrichtung (116) umfasst, die dazu ausgestaltet ist, eine oder mehrere Eingaben von der Kartuschenidentifikationsschaltungsanordnung (174) zu empfangen und die eine oder mehreren Eingaben, die eine der auswechselbaren Kartusche zugehörige Identifikation (172) angeben, zum Empfang durch die Kartuschenidentifikationsschaltungsanordnung (174) zu erzeugen.

8. System (100) nach Anspruch 7, wobei die Identifizierungseinrichtung (116) einen Abschnitt umfasst, der dazu ausgestaltet ist, mit der Kartuschenidentifikationsschaltungsanordnung (174) gekoppelt zu werden, und der einen elektrischen Widerstand aufweist, der die auswechselbare Kartusche angibt.

9. System (100) nach Anspruch 7, wobei die Identifizierungseinrichtung (116) ein oder mehrere mechanische Merkmale umfasst, welche die auswechselbare Kartusche angeben und die dazu ausgestaltet sind, mit einem oder mehreren Schaltern in Eingriff zu gelangen, die in der Kartuschenidentifikationsschaltungsanordnung (174) enthalten sind.

10. System (100) nach Anspruch 6, wobei die auswechselbare Kartusche (110) eine erste auswechselbare Kartusche ist, wobei das System ferner Folgendes umfasst: eine zweite auswechselbare Kartusche (110), die ein oder mehrere Fluidreservoire (114) umfasst und dazu ausgestaltet ist, durch den Vernebelungskörper (120) lösbar aufgenommen zu werden; ein Ventil, das in Fluidverbindung mit dem einen oder den mehreren Fluidreservoiren (114) steht; und eine zweite Identifizierungseinrichtung (116), die dazu ausgestaltet ist, eine oder mehrere Eingaben zu erzeugen, die eine der zweiten auswechselbaren Kartusche zugehörige Identifikation (172) angeben,
wobei die eine oder mehreren Eingaben, die eine der zweiten auswechselbaren Kartusche zugehörige Identifikation (172) angeben, einem oder mehreren Fluiden entsprechen, die in dem einen oder den mehreren Fluidreservoiren (114) der zweiten auswechselbaren Kartusche (110) angeordnet sind, und wobei die Abgabeaerosolschaltungsanordnung (178) dazu ausgestaltet ist, das wenigstens eine elektromechanische Vernebelungselement (123) basierend auf dem einen oder den mehreren Fluiden, die in dem einen oder den mehreren Fluidreservoiren (114) der zweiten auswechselbaren Kartusche (110) angeordnet sind, zu betreiben.

11. System (100) nach Anspruch 1, wobei die auswechselbare Kartusche (110) eine kosmetische oder dermatologische Zusammensetzung umfasst, die in dem einen oder den mehreren Fluidreservoiren (114) angeordnet ist.

12. System (100) nach Anspruch 1, wobei das wenigstens eine elektromechanische Vernebelungselement (123) ein piezoelektrisches Element umfasst, das dazu ausgestaltet ist, in Fluidverbindung mit dem einen oder den mehreren Fluidreservoiren (114) der auswechselbaren Kartusche (110) zu stehen, wenn das Ventil in einem offenen Zustand ist.

13. System (100) nach Anspruch 12, wobei das wenigstens eine elektromechanische Vernebelungselement (123) ferner ein Netz (125) umfasst, das dem piezoelektrischen Element benachbart angeordnet und von dem piezoelektrischen Element durch einen Raum getrennt ist und das dazu ausgestaltet ist, ein Fluid aus dem einen oder den mehreren Fluidreservoiren (114) der auswechselbaren Kartusche (110) zu empfangen.

14. System (100) nach Anspruch 1, wobei das wenigstens eine elektromechanische Vernebelungselement (123) dazu ausgestaltet ist, ein Aerosol abzugeben, das Partikel mit einem mittleren Durchmesser von weniger als 100 µm umfasst.

15. System (100) nach Anspruch 12, das ferner eine Abgabeaerosolschaltungsanordnung (178) umfasst, die mit dem wenigstens einen elektromechanischen Vernebelungselement (123), welches das piezoelektrische Element umfasst, wirkgekoppelt ist und dazu ausgestaltet ist, ein elektrisches Feld an das piezoelektrische Element anzulegen, wobei das elektrische Feld eine Frequenz und eine Amplitude aufweist, die auf eine oder mehrere Eingaben, die eine der auswechselbaren Kartusche zugehörige Identifikation (172) angeben, reagieren.

## Revendications

1. Système (100) comprenant :
un corps de nébulisation (120) incluant au moins un élément de nébulisation électromécanique (123) ; et
une cartouche remplaçable (110) incluant un ou plusieurs réservoirs de fluide (114) et une vanne en communication fluidique avec les un ou plusieurs réservoirs de fluide (114),
dans lequel le corps de nébulisation (120) est configuré pour recevoir, de manière libérable, la cartouche remplaçable, **caractérisé en ce que**
le corps de nébulisation (120) inclut un commutateur configuré pour mettre en prise sélectivement la cartouche remplaçable (110) lorsque la cartouche remplaçable est reçue, de manière libérable, par le corps de nébulisation (120), en plaçant de ce fait la vanne dans un état ouvert.

2. Système (100) selon la revendication 1, dans lequel la vanne est dans un état ouvert et les un ou plusieurs réservoirs de fluide (114) sont couplés en communication fluidique à l'au moins un élément de nébulisation électromécanique (123) lorsque la cartouche remplaçable (110) est reçue par le corps de nébulisation (120) et est en prise avec celui-ci.

3. Système (100) selon la revendication 1, dans lequel la vanne est dans un état fermé et les un ou plusieurs réservoirs de fluide (114) ne sont pas couplés en communication fluidique à l'au moins un élément de nébulisation électromécanique (123) dans une configuration hors prise.

4. Système (100) selon la revendication 1, dans lequel le corps de nébulisation (120) est configuré pour recevoir, de manière libérable, la cartouche remplaçable (110) sans mise en prise avec la cartouche remplaçable.

5. Système (100) selon la revendication 1, dans lequel le corps de nébulisation (120) et la cartouche remplaçable (110) sont configurés en coopération de sorte que la cartouche remplaçable puisse être reçue de manière libérable par le corps de nébulisation (120) et mise en prise avec celui-ci après avoir été précédemment reçue de manière libérable par le corps de nébulisation (120) et mise en prise avec celui-ci.

6. Système (100) selon la revendication 1, comprenant en outre une circuiterie d'aérosol de décharge (178) couplée fonctionnellement à l'au moins un élément de nébulisation électromécanique (123) et configurée pour générer un aérosol de décharge en réponse à une ou plusieurs entrées indicatives d'une identification de cartouche remplaçable (172), dans lequel les une ou plusieurs entrées indicatives d'une identification de cartouche remplaçable (172) correspondent à un ou plusieurs fluides disposés dans les un ou plusieurs réservoirs de fluide (114), et dans lequel la circuiterie d'aérosol de décharge (178) est configurée pour actionner l'au moins un élément de nébulisation électromécanique (123) sur la base des un ou plusieurs fluides disposés dans les un ou plusieurs réservoirs de fluide (114).

7. Système (100) selon la revendication 6, comprenant en outre une circuiterie d'identification de cartouche (178) couplée fonctionnellement à la circuiterie d'aérosol de décharge (178), la circuiterie d'identification de cartouche (174) étant configurée pour recevoir une ou plusieurs entrées depuis un identifiant (116) porté par la cartouche remplaçable et pour générer les une ou plusieurs entrées indicatives d'une identification de cartouche remplaçable (174) destinées à être reçues par la circuiterie de décharge d'aérosol (178),
dans lequel la circuiterie d'identification de cartouche (174) inclut l'un parmi un lecteur d'un dispositif d'identification par radiofréquences et d'un initiateur de communication en champ proche,
dans lequel la cartouche remplaçable inclut un identifiant (116) configuré pour recevoir une ou plusieurs entrées depuis la circuiterie d'identification de cartouche (174) et générer les une ou plusieurs entrées indicatives d'une identification de cartouche remplaçable (172) destinées à être reçues par la circuiterie d'identification de cartouche (174).

8. Système (100) selon la revendication 7, dans lequel l'identifiant (116) inclut une partie configurée pour se coupler à la circuiterie d'identification de cartouche (174) ayant une résistance électrique indicative de la cartouche remplaçable.

9. Système (100) selon la revendication 7, dans lequel l'identifiant (116) inclut une ou plusieurs caractéristiques mécaniques indicatives de la cartouche remplaçable configurées pour se mettre en prise avec un ou plusieurs commutateurs inclus dans la circuiterie d'identification de cartouche (174).

10. Système (100) selon la revendication 6, dans lequel la cartouche remplaçable (110) est une première cartouche remplaçable, le système comprenant en outre une seconde cartouche remplaçable (110) incluant un ou plusieurs réservoirs de fluide (114) et configurée pour être reçue de manière libérable par le corps de nébulisation (120) ; une vanne en communication fluidique avec les un ou plusieurs réservoirs de fluide (114) ; et un second identifiant (116) configuré pour générer une ou plusieurs entrées indicatives d'une seconde identification de cartouche remplaçable (172),
dans lequel les une ou plusieurs entrées indicatives d'une seconde identification de cartouche remplaçable (172) correspondent à un ou plusieurs fluides disposés dans les un ou plusieurs réservoirs de fluide (114) de la seconde cartouche remplaçable (110), et dans lequel la circuiterie d'aérosol de décharge (178) est configurée pour actionner l'au moins un élément de nébulisation électromécanique (123) sur la base des un ou plusieurs fluides disposés dans les un ou plusieurs réservoirs de fluide (114) de la seconde cartouche remplaçable (110).

11. Système (100) selon la revendication 1, dans lequel la cartouche remplaçable (110) inclut une composition cosmétique ou dermatologique disposée dans les un ou plusieurs réservoirs de fluide (114).

12. Système (100) selon la revendication 1, dans lequel l'au moins un élément de nébulisation électromécanique (123) inclut un élément piézoélectrique configuré pour être en communication fluidique avec les un ou plusieurs réservoirs de fluide (114) de la cartouche remplaçable (110) lorsque la vanne est dans un état ouvert.

13. Système (100) selon la revendication 12, dans lequel l'au moins un élément de nébulisation électromécanique (123) inclut en outre une maille (125) disposée adjacente à l'élément piézoélectrique et séparée de l'élément piézoélectrique par un espace configuré pour recevoir un fluide depuis les un ou plusieurs réservoirs de fluide (114) de la cartouche remplaçable (110).

14. Système (100) selon la revendication 1, dans lequel l'au moins un élément de nébulisation électromécanique (123) est configuré pour décharger un aérosol incluant des particules présentant un diamètre moyen inférieur à 100 µm.

15. Système (100) selon la revendication 12, comprenant en outre une circuiterie d'aérosol de décharge (178) couplée fonctionnellement à l'au moins un élément de nébulisation électromécanique (123) incluant l'élément piézoélectrique et configurée pour appliquer un champ électrique à l'élément piézoélectrique, le champ électrique présentant une fréquence et une amplitude réactives à une ou plusieurs entrées indicatives d'une identification de cartouche remplaçable (172).
